# EUROPÄISCHE PATENTANMELDUNG

(11) **EP 4 000 495 A1**
(43) Veröffentlichungstag der Anmeldung: **25.05.2022**
(21) Anmeldenummer: 21207709.3
(22) Anmeldetag: 11.11.2021
(51) Int. Cl.: A61B 1/00, A61B 90/50

(54) **HALTEVORRICHTUNG, MEDIZINISCHES SYSTEM UND VERFAHREN ZUR POSITIONIERUNG EINES MEDIZINISCHEN INSTRUMENTS**

(30) Priorität: 18.11.2020 DE 102020130493
(71) Anmelder: Karl Storz SE & Co. KG, 78532 Tuttlingen (DE)
(72) Erfinder: HUBER, Florian, 78532 Tuttlingen (DE); LETTNER, Barbara, 78532 Tuttlingen (DE); GLÖGGLER, Bernhard, 78532 Tuttlingen (DE)

(57) **Zusammenfassung**

Die Offenbarung betrifft eine Haltevorrichtung (12) für medizinische Instrumente (14), mit einer proximalen Basis (20) zur Aufnahme an einem Gestell (16), die ein erstes Gelenk (30) umfasst, einem distalen Instrumentenhalter (22), der ein zweites Gelenk (32) umfasst, zumindest zwei Schubelementen (52, 54, 58) zwischen der Basis (20) und dem Instrumentenhalter (22), und zumindest zwei Klemmelementen (138, 140, 142, 144), die durch zumindest ein Übertragungselement (84, 86, 88) betätigbar sind, die gemeinsam in einem Klemmzustand und einem Freigabezustand betreibbar sind, und die dazu ausgebildet sind, Gelenke der Haltevorrichtung (12) im Klemmzustand zu blockieren, wobei die zumindest zwei Schubelemente (52, 54, 58) ein Schubgelenk (24) bilden und relativ zueinander entlang einer Längsachse (132) translatorisch verfahrbar sind, und wobei die zumindest zwei Schubelemente (52, 54, 58) relativ zueinander teleskopierbar und drehfest miteinander gekoppelt sind. Die Offenbarung betrifft ferner ein medizinisches System, eine Verwendung einer Haltevorrichtung, sowie ein Verfahren zur Positionierung eines medizinischen Instruments.

## Beschreibung

Die vorliegende Offenbarung bezieht sich auf eine Haltevorrichtung für medizinische Instrumente, insbesondere endoskopische Instrumente.

Aus der DE 103 07 054 A1 ist ein Halte- und Führungssystem für ein medizinisches Instrument, insbesondere ein Endoskop, bekannt, mit zumindest einem Führungsarm, an dem das medizinische Instrument angekoppelt ist, wobei der Führungsarm unter Ausbildung eines kinematischen Dreibeins mittels zweier Haltestangen beweglich gelagert ist.

Im medizinischen Bereich sind Instrumente etabliert, die handgehalten und/oder handgeführt genutzt werden. Hierbei handelt sich beispielsweise um handgeführte endoskopische Instrumente, die durch einen Trokar in das Körperinnere eines Patienten einführbar sind. Es sind jedoch auch Instrumente bekannt, beispielsweise Beobachtungsinstrumente, die in Bezug auf den Patienten positioniert werden und dann zumindest einige Zeit fixiert bleiben. Mischformen sind vorstellbar, umfassend Instrumente die von Zeit zu Zeit während eines Eingriffs neu positioniert werden.

Medizinische Instrumente, gerade auch handgeführte endoskopische Instrumente, werden zuweilen an Stativen oder dergleichen aufgenommen und in Bezug auf den Patienten positioniert und ausgerichtet. Ein Vorteil dieses Ansatzes besteht darin, dass das medizinische Fachpersonal (zum Beispiel der Operateur) danach beide Hände frei hat. Gestelle und Stative für medizinische Instrumente können jedoch recht komplex und aufwendig gestaltet sein und eine Mehrzahl von Bedienelementen umfassen. Dies führt dazu, dass eine Verstellung häufig mit einem hohen Aufwand verbunden ist. Ferner ist zu beachten, dass medizinische Prozeduren häufig unter erhöhten Anforderungen an Hygiene durchgeführt werden (Reinraumbedingungen, Sterilisation, etc.).

Im Stand der Technik sind auch sogenannte Operationsroboter beschrieben, die sämtliche Freiheitsgrade eines medizinischen Instruments über eine Bedienkonsole motorisch steuern können. Mit anderen Worten muss der Operateur nicht mehr unmittelbar beim Patienten agieren. Operationsroboter sind sehr kostenintensiv und kommen bisher in der Praxis nur begrenzt zum Einsatz.

Für bestimmte Anwendungen ist es im Übrigen gar nicht unbedingt erforderlich, eine Vielzahl von Bewegungsfreiheitsgraden für gehaltene Instrumente bereitzustellen. Häufig genügt es, ein aufgenommenes Instrument, etwa ein endoskopisches Instrument, im Wesentlichen frei in einer bestimmten Ebene positionieren zu können, und an der gewünschten Position bei Bedarf einen oder mehrere Schwenkfreiheitsgrade für das Instrument bereitzustellen. Die Kraft zur Bewegung und Ausrichtung des Instruments kann durch das medizinische Fachpersonal aufgebracht werden, zumindest in bestimmten Anwendungen. In einem solchen Fall kann das Gestell/Stativ gesperrt werden, wenn die gewünschte Position erreicht ist.

Bei medizinischen Prozeduren, insbesondere bei Operationen und anderen chirurgischen Eingriffen, ist ein freies Sichtfeld und die freie Erreichbarkeit des Patienten von hoher Bedeutung für das medizinische Fachpersonal. Daher ist es generell wünschenswert, den unmittelbaren Operationsbereich nicht mit sperrigen, ausladenden Gerätschaften zu versperren bzw. zu verbauen.

Vor diesem Hintergrund liegt der vorliegenden Offenbarung die Aufgabe zugrunde, eine Haltevorrichtung für medizinische Instrumente anzugeben, die die Aufnahme und Positionierung eines medizinischen Instruments erlaubt und zu diesem Zweck geeignete Freiheitsgrade bereitstellt. Insbesondere soll die Haltevorrichtung die Positionierung eines medizinischen Instruments in einer Ebene ermöglichen und dort zumindest eine Schwenkachse für das Instrument bereitstellen.

Die Haltevorrichtung soll einfach bedienbar sein. Die Haltevorrichtung soll eine manuelle Positionierung des Instruments ermöglichen, zumindest in beispielhaften Ausgestaltungen. Um das positionierte Instrument zu fixieren, soll die Haltevorrichtung mit geringem Aufwand gesperrt werden können, zumindest in beispielhaften Ausgestaltungen. In ähnlicher Weise ist es bevorzugt, wenn die Haltevorrichtung einfach entsperrt werden kann, um das Instrument neu zu positionieren bzw. auszurichten.

Ferner sollen im Rahmen der vorliegenden Offenbarung ein medizinisches System mit einer derartigen Haltevorrichtung, eine korrespondierende Verwendung einer Haltevorrichtung zur Positionierung eines medizinischen Instruments sowie ein korrespondierendes Verfahren zur Positionierung eines medizinischen Instruments angegeben werden.

Gemäß einem ersten Aspekt bezieht sich die vorliegende Offenbarung auf eine Haltevorrichtung für medizinische Instrumente, insbesondere endoskopische Instrumente, die Folgendes aufweist:
- eine proximale Basis zur Aufnahme an einem Gestell, die ein erstes Gelenk umfasst,
- einen distalen Instrumentenhalter, der ein zweites Gelenk umfasst,
- zumindest zwei Schubelemente zwischen der Basis und dem Instrumentenhalter, und
- zumindest zwei Klemmelemente, die durch zumindest ein Übertragungselement betätigbar sind, die gemeinsam in einem Klemmzustand und einem Freigabezustand betreibbar sind, und die dazu ausgebildet sind, Gelenke der Haltevorrichtung im Klemmzustand zu blockieren,
- wobei die zumindest zwei Schubelemente ein Schubgelenk bilden und relativ zueinander entlang einer Längsachse translatorisch verfahrbar sind, und
- wobei die zumindest zwei Schubelemente relativ zueinander teleskopierbar und drehfest miteinander gekoppelt sind.

Auf diese Weise wird die Aufgabe gelöst.

Erfindungsgemäß wird nämlich ein Mechanismus bereitgestellt, der mehrere Bewegungsfreiheitsgrade bereitstellt, die gemeinsam gesperrt oder freigegeben werden können. Die Bedienung vereinfacht sich. Der Aufwand für die Positionierung und Ausrichtung des Instruments kann sinken. Die Haltevorrichtung ist kompakt und platzsparend gestaltet.

Bei den beweglichen Gelenken handelt sich beispielsweise um Glieder des ersten Gelenks, des zweiten Gelenks und/oder des Schubgelenks. Gemeinsam bedeutet, dass mehrere oder sämtliche der Klemmelemente gleichzeitig angesteuert werden können, um diese bedarfsweise in den Klemmzustand oder den Freigabezustand zu bringen, zumindest in beispielhaften Ausgestaltungen.

Endoskopische Instrumente sind Instrumente, die dazu ausgebildet sind, durch natürliche oder künstlich geschaffene Öffnungen in den Körper eines Patienten eingeführt zu werden. Endoskopische Instrumente umfassen beispielsweise auch laparoskopische Instrumente, neurochirurgische Instrumente und dergleichen.

Im Sinne der vorliegenden Offenbarung handelt es sich bei einem distalen Element um ein Element, das aus Sicht einer Basis oder eines Gestells der Haltevorrichtung dem Patienten bzw. der Patientenauflage zugewandt bzw. näher beim Patienten angeordnet ist als ein proximales Element. Im Sinne der vorliegenden Offenbarung handelt es sich bei einem proximalen Element um ein Element, das aus Sicht des Patienten bzw. der Patientenauflage dem Gestell zugewandt bzw. näher beim Gestell angeordnet ist als ein distales Element. Allgemein werden daher im Rahmen der vorliegenden Offenbarung die Begriffe proximal und distal im Rahmen ihrer üblichen Bedeutung auf dem Gebiet der Medizintechnik verwendet. Dies ist nicht einschränkend zu verstehen.

Teleskopierbar umfasst im Sinne der vorliegenden Offenbarung beispielsweise eine ineinander verfahrbare Gestaltung der zumindest zwei Schubelemente. Ein teleskopierbares Schubgelenk ist zwischen einer eingefahrenen Stellung und einer ausgefahrenen Stellung verfahrbar. Beispielsweise ist zumindest ein Klemmelement vorgesehen, um die aktuelle Stellung des Schubgelenks bedarfsweise zu blockieren.

Im Klemmzustand hemmen oder sperren die Klemmelemente Relativbewegungen zwischen benachbarten Elementen, die im Freigabezustand relativ zueinander beweglich sind. Es ist nicht unbedingt eine formschlüssige Lagesicherung erforderlich, zumindest in beispielhaften Ausgestaltungen. In einer beispielhaften Ausgestaltung sind die Klemmelemente dazu ausgestaltet, die Reibung zwischen den benachbarten Elementen derart zu erhöhen, dass eine Relativbewegung zwischen diesen Elementen nur mit entsprechend hohem Kraftaufwand möglich ist. Auf diese Weise kann eine Klemmung/Sperrung herbeigeführt werden. Wenn die Haltevorrichtung im Klemmzustand blockiert ist, ist das medizinische Instrument hinreichend fest am Instrumentenhalter aufgenommen.

Gemäß einer beispielhaften Ausgestaltung ist das erste Gelenk als Drehgelenk gestaltet. Gemäß einer weiteren beispielhaften Ausgestaltung ist das zweite Gelenk als Kugelgelenk gestaltet. Auf diese Weise gibt es neben dem Schubgelenk noch ein Drehgelenk und gegebenenfalls ein Kugelgelenk. Gemeinsam sorgen das Schubgelenk und das Drehgelenk dafür, dass die Haltevorrichtung das Instrument in einer Ebene platzieren kann, beispielsweise parallel zur Patientenauflage. Der anfahrbare Bereich ergibt sich aus dem möglichen Verfahrweg des Schubgelenks sowie dem möglichen Schwenkwinkel des Drehgelenks. Das Kugelgelenk erlaubt Schwenkbewegungen des Instruments um den Instrumentenhalter, zumindest in beispielhaften Ausgestaltungen.

Es versteht sich, dass im Rahmen der vorliegenden Offenbarung die Bezeichnungen erstes Element, zweites Element und dergleichen keine zwingende Ordnung oder Gewichtung definieren, sondern vorrangig der Unterscheidbarkeit dienen. Daher sind grundsätzlich auch Ausführungsbeispiele vorstellbar, die nur eines der Elemente aufweisen, beispielsweise nur das zweite Element.

Gemäß einer beispielhaften Ausgestaltung ist die Haltevorrichtung dazu ausgebildet, Bewegungen in einer Ebene, insbesondere in einer Ebene, die parallel zu einer Patientenauflage ausgerichtet ist, zu ermöglichen. Auf diese Weise kann das Instrument an einem gewünschten Ort positioniert und dort unter Nutzung des Kugelgelenks in einer gewünschten Orientierung festgelegt werden. In einer beispielhaften Ausgestaltung ist das Instrument zusätzlich entlang seiner Längsachse im Kugelgelenk translatorisch verfahrbar und positionierbar.

Gemäß einer beispielhaften Ausgestaltung ist zwischen dem ersten Gelenk bei der proximalen Basis und einem proximalen Schubelement ein Klemmelement vorgesehen, das auf das erste Gelenk einwirkt, wobei zwischen dem zweiten Gelenk bei dem distalen Instrumentenhalter ein Klemmelement vorgesehen ist, das auf das zweite Gelenk einwirkt.

Gemäß einer weiteren beispielhaften Ausgestaltung umfasst das erste Gelenk eine Aufnahme und ein Schwenkstück, die gemeinsam eine Schwenkachse definieren, wobei dem ersten Gelenk ein proximales Klemmelement zugeordnet ist, das das erste Gelenk bedarfsweise im Klemmzustand hemmt oder sperrt. Auf diese Weise kann ein gegebener Schwenkzustand des Instrumentenhalters zur Lagesicherung fixiert werden. Vorzugsweise geht dies einher mit einer Lagesicherung des Schubgelenks in der gegebenen Ausfahrposition.

Gemäß einer beispielhaften Ausgestaltung umfasst das Klemmelement für das erste Gelenk einen Exzenter, der als proximaler Fortsatz über das proximale Schubelement hervorsteht. Der Exzenter ist mit einem Übertragungselement gekoppelt. Gemäß einer beispielhaften Ausgestaltung erstreckt sich der Exzenter in eine Ausnehmung im Schwenkstück, wobei der Exzenter im Klemmzustand derart in der Ausnehmung rotiert ist und auf einen Stempel bzw. ein Druckstück einwirkt, dass das Schwenkstück und die Aufnahme miteinander verspannt und blockiert sind.

Gemäß einer weiteren beispielhaften Ausgestaltung umfasst das zweite Gelenk eine Führungskugel und eine Kugelpfanne, insbesondere eine jochartig gestaltete Kugelpfanne, wobei die Führungskugel eine Instrumentenaufnahme zur Aufnahme des medizinischen Instruments aufweist, und wobei dem zweiten Gelenk ein distales Klemmelement zugeordnet ist, das das zweite Gelenk bedarfsweise im Klemmzustand hemmt oder sperrt.

Auf diese Weise kann das zweite Gelenk mehrere Schwenkfreiheitsgrade bereitstellen. Diese Bewegungsfreiheitsgrade können mit einem Klemmelement bedarfsweise gehemmt/gesperrt werden. Die Führungskugel umfasst beispielsweise eine Ausnehmung, die einen Schaft des Instruments aufnehmen kann. Auf diese Weise kann bedarfsweise noch ein Rotationsfreiheitsgrad für Rotation des Instruments um dessen Schaft bereitgestellt werden, der ebenso mit dem Klemmelement gehemmt/gesperrt werden kann. Beispielhaft umfasst die Führungskugel ein hinreichend elastisches Material.

Gemäß einer weiteren beispielhaften Ausgestaltung wirkt das distale Klemmelement auf die jochartig gestaltete Kugelpfanne ein, um die Führungskugel relativ zur Kugelpfanne festzulegen. Auf diese Weise werden die Freiheitsgrade des Kugelgelenks gesperrt.

Gemäß einer beispielhaften Ausgestaltung umfasst das Klemmelement für das zweite Gelenk einen nach innen wirkenden Klemmring, der als distaler Abschluss eines distalen Übertragungselements gestaltet ist und von außen auf zwei Schenkel der jochartig gestalteten Kugelpfanne einwirkt, um die beiden Schenkel zusammenzudrücken. Auf diese Weise wird die Führungskugel in der Kugelpfanne verspannt oder blockiert. Der Klemmring kann auch als Exzenterring bezeichnet werden. Der Klemmring ist mit einer Exzenterkontur versehen, um im Klemmzustand die beiden Schenkel zu belasten, um die Führungskugel zu blockieren.

In einer beispielhaften Ausgestaltung ist ein distaler Abschnitt des ersten Gelenks, also beispielsweise das Schwenkstück des Drehgelenks, am proximalen Schubelement drehfest befestigt. In einer beispielhaften Ausgestaltung ist ein proximaler Abschnitt des zweiten Gelenks, also beispielsweise die Pfanne des Kugelgelenks, am distalen Schubelement drehfest befestigt. Auf diese Weise ergibt sich eine geschlossene, integrierte Gestaltung. Verschiedene Elemente werden durch die Schubelemente abgedeckt und geschützt.

Gemäß einer weiteren beispielhaften Ausgestaltung ist ein gemeinsamer Antrieb für die zumindest zwei Klemmelemente vorgesehen, der die zumindest zwei Klemmelemente simultan betätigt. Gemäß einer weiteren beispielhaften Ausgestaltung ist ein einziger Antrieb vorgesehen. In einer beispielhaften Ausgestaltung ist ein einziger Antrieb vorgesehen, der sämtliche Klemmelemente simultan betätigt. In einer beispielhaften Ausgestaltung werden die Klemmelemente synchron/gleichzeitig betätigt

In einer beispielhaften Ausgestaltung handelt es sich bei dem Antrieb um einen manuell zu betätigenden Antrieb. In einer beispielhaften Ausgestaltung handelt es sich bei dem Antrieb um einen motorischen Antrieb, insbesondere um einen elektromotorischen Antrieb. Es versteht sich, dass grundsätzlich auch ein pneumatischer oder fluidischer Antrieb den motorischen Antrieb bilden kann. In einer beispielhaften Ausgestaltung ist ein Schalter zur Betätigung des Motors vorgesehen. Der Schalter kann unmittelbar bei der Haltevorrichtung angeordnet sein. Der Schalter kann jedoch auch von der Haltevorrichtung beabstandet sein. In einer beispielhaften Ausgestaltung weist der Schalter zumindest zwei Zustände auf, die dem Freigabezustand und dem Klemmzustand entsprechen. In einer beispielhaften Ausgestaltung weist der Schalter zumindest drei Zustände auf, die eine Aktivierung des Antriebs in einer ersten Drehrichtung mit erstem Drehsinn, eine Aktivierung des Antriebs in einer gegensinnigen zweiten Drehrichtung und eine Deaktivierung des Antriebs betreffen.

In einer beispielhaften Ausgestaltung umfasst der Antrieb einen Motor, der bei einem der Schubelemente angeordnet ist. In einer beispielhaften Ausgestaltung ist der Motor bei einem proximalen Schubelement angeordnet, das dem ersten Gelenk benachbart ist, wobei sich an das proximale Schubelement zumindest ein distales Schubelement anschließt. Dies führt zu einer günstigen Massenverteilung bzw. Massenträgheit, zumindest in beispielhaften Ausgestaltungen. Bauteile der Haltevorrichtung, die größer und/oder schwerer sind, sind näher bei der proximalen Basis als bei dem distalen Instrumentenhalter vorgesehen.

Gemäß einer weiteren beispielhaften Ausgestaltung umfasst der Antrieb eine Antriebswelle, die mit zumindest einem der Klemmelemente zusammenwirkt, wobei der Antrieb ferner ein Lagerstück aufweist, das beim Antreiben der Antriebswelle ein resultierendes Gegenmoment aufnimmt, und wobei das Lagerstück mit zumindest einem anderen der Klemmelemente zusammenwirkt. Mit anderen Worten können also das Antriebsmoment und das resultierende Gegenmoment, mit dem sich ein am Gestell festgelegter Antrieb am Gestell abstützen würde, genutzt werden, um Schubelemente gegenläufig anzutreiben.

In einer beispielhaften Ausgestaltung ist der Antrieb "schwimmend" innerhalb der zumindest zwei Schubelemente gelagert. Mit anderen Worten ist der Antrieb nicht notwendigerweise an einem Gestell oder einer Basis festgelegt. Dies erfolgt allenfalls mittelbar.

Gemäß einer weiteren beispielhaften Ausgestaltung ist die Antriebswelle parallel zur Längsachse orientiert, wobei das Lagerstück mit einem proximalen Klemmelement gekoppelt ist, das dem ersten Gelenk zugeordnet ist. Die Antriebswelle erstreckt sich in einer beispielhaften Ausgestaltung in Richtung auf den distalen Instrumentenhalter. Die Kopplung des Lagerstücks mit dem proximalen Klemmelement kann direkt oder mittelbar erfolgen.

Gemäß einer weiteren beispielhaften Ausgestaltung ist der Antrieb mit zumindest zwei teleskopierbaren Übertragungselementen gekoppelt, wobei die Anzahl der Übertragungselemente an die Anzahl der Schubelemente angepasst ist, und wobei die Übertragungselemente eine durch den Antrieb induzierte Drehbewegung übertragen, um die zumindest zwei Klemmelemente in den Klemmzustand oder den Freigabezustand zu überführen. In einer beispielhaften Ausgestaltung ist jeweils die gleiche Anzahl Schubelemente und Übertragungselemente vorgesehen. Die Übertragungselemente können auch als Kraftübertragungselemente bezeichnet werden und dienen der Übertragung der Antriebsbewegung bzw. des Antriebsmoments.

In einer beispielhaften Ausgestaltung sind die Schubelemente und die Übertragungselemente gemeinsam simultan teleskopierbar. Mit anderen Worten kann ein Bediener die Schubelemente bewegen, um die Haltevorrichtung auszufahren. Bei der translatorischen Bewegung der Schubelemente werden auch die Übertragungselemente translatorisch bewegt. Mit anderen Worten erfolgt eine Mitnahme der Übertragungselemente durch die Schubelemente bei der translatorischen Bewegung, zumindest in beispielhaften Ausgestaltungen.

In einer beispielhaften Ausgestaltung wird die Teleskopierbewegung manuell erzeugt, wenn sich die Klemmelemente im Freigabezustand befinden.

In einer beispielhaften Ausgestaltung sind die Übertragungselemente innerhalb der Schubelemente angeordnet. In einer beispielhaften Ausgestaltung handelt es sich bei den Übertragungselementen um Rohrstücke, insbesondere um zylindrische Rohrstücke mit einem Ringprofil. In einer beispielhaften Ausgestaltung sind die Querschnitte der Übertragungselemente derart aneinander angepasst, dass die Übertragungselemente teleskopiert werden können.

In einer beispielhaften Ausgestaltung bilden die Schubelemente eine Außenschale mit Drehsicherung, wobei die Übertragungselemente eine zumindest teilweise rotierbar Innenschale bilden.

Gemäß einer weiteren beispielhaften Ausgestaltung ist der Antrieb derart mit zwei benachbarten Übertragungselementen gekoppelt, dass die benachbarten Übertragungselemente bei einer Bewegung des Antriebs gegensinnig rotiert werden. Damit kann sich der Antrieb im Falle einer "schwimmenden" Lagerung an den gegensinnig rotierbaren Übertragungselementen abstützen, wobei ein Übertragungselement über die (abriebseitige) Antriebswelle und ein anderes Übertragungselement über das (mit dem Antriebsgehäuse gekoppelte) Lagerstück angetrieben wird.

In einer beispielhaften Ausgestaltung ist das Lagerstück des Antriebs mit einem ersten Übertragungselement gekoppelt, wobei die Antriebswelle mit einem benachbarten zweiten Übertragungselement gekoppelt ist. Auf diese Weise wird das Antriebsmoment einerseits in das zweite Übertragungselement eingeleitet. Das Gegenmoment wird nicht in ein gestellfestes Teil, sondern in das erste Übertragungselement eingeleitet. Auf diese Weise werden die beiden Übertragungselemente gegensinnig rotiert.

In einer beispielhaften Ausgestaltung ist daher der Antrieb nicht gestellfest gelagert, sondern mittelbar mit der Basis gekoppelt. Auf diese Weise kann das Gegenmoment auch zur Bewegungserzeugung genutzt werden. In einer beispielhaften Ausgestaltung verspannt der Antrieb zwei benachbarte Übertragungselemente gegeneinander.

Gemäß einer weiteren beispielhaften Ausgestaltung sind die Schubelemente und die Übertragungselemente jeweils als Hohlprofilkörper gestaltet, wobei die Übertragungselemente innerhalb der Schubelemente angeordnet sind, und wobei der Antrieb ein patronenartiges Gehäuse aufweist, das drehfest mit einem der Übertragungselemente gekoppelt ist, insbesondere mit einem proximalen Übertragungselement. Auf diese Weise ergibt sich eine integrierte Gestaltung. Verschiedene bewegliche Komponenten der Haltevorrichtung sind durch die Schubelemente geschützt.

Gemäß einer weiteren beispielhaften Ausgestaltung sind drei oder mehr Übertragungselemente vorgesehen, von denen zumindest zwei benachbarte Übertragungselemente, insbesondere zwei distale Übertragungselemente, drehfest miteinander gekoppelt und relativ zueinander translatorisch verfahrbar sind. Auf diese Weise können die Übertragungselemente die Teleskopierbewegung der Schubelemente mitmachen und gleichwohl dazu beitragen, die Schubelemente mit nur einem Antrieb bzw. einer Antriebsbewegung simultan zu sperren oder freizugeben.

In einer beispielhaften Ausgestaltung weist eines der beiden Übertragungselemente zumindest eine sich axial erstreckende Führung auf, beispielsweise zumindest ein Langloch, wobei das andere der beiden Übertragungselemente ein korrespondierendes Führungselement aufweist, beispielsweise einen Führungsbolzen oder Führungsstift, wobei das Führungselement in der Führung beweglich gelagert ist.

Gemäß einer weiteren beispielhaften Ausgestaltung umfasst die Haltevorrichtung zumindest drei Schubelemente, die ein Schubgelenk bilden und teleskopierbar sind, wobei Querschnittsprofile der Schubelemente ausgehend von der proximalen Basis hin zum distalen Instrumentenhalter abgestuft sind und kleiner werden. Dies ist günstig für die Steifigkeit, Massenträgheit und die Massenverteilung.

Gemäß einer weiteren beispielhaften Ausgestaltung weisen die zumindest zwei Schubelemente ein Kastenprofil auf, insbesondere ein Vierkantprofil, und wobei die Kastenprofile der Schubelemente derart aneinander angepasst sind, dass die Schubelemente entlang der Längsachse teleskopierbar und verdrehsicher miteinander gekoppelt sind. Das Querschnittsprofil der Schubelemente kann beispielsweise ein Vierkantprofil in Form eines Rechteckprofils oder Quadratprofils sein. Andere Gestaltungen sind grundsätzlich denkbar. Beispielhafte Gestaltungen umfassen Querschnittsprofile, die unrund sind, also eine verdrehsichere Form aufweisen.

Gemäß einer weiteren beispielhaften Ausgestaltung ist zwischen zwei benachbarten Schubelementen jeweils ein Klemmelement vorgesehen. Es kann sich um direkt auf die Schubelemente wirkende Klemmelemente handeln. Es ist jedoch auch vorstellbar, Klemmelemente vorzusehen, die mittelbar/indirekt wirken, um jeweils zwei benachbarte Schubelemente relativ zueinander zu sperren/fixieren. Dies kann beispielsweise unter Zwischenschaltung der Übertragungselemente erfolgen.

Gemäß einer weiteren beispielhaften Ausgestaltung sind zumindest die den Schubelementen zugeordneten Klemmelemente rotatorisch betätigbar, wobei die entsprechenden Klemmelemente die Schubelemente mit dem zumindest einen Übertragungselement in einer gegebenen translatorischen Position klemmen.

Gemäß einer weiteren beispielhaften Ausgestaltung weist zumindest eines der Klemmelemente einen Exzenter auf, wobei zumindest ein Exzenter als Federexzenter gestaltet ist und/oder Exzenterkonturen aufweist, und wobei der Exzenter bei einer Relativrotation zwischen dem zumindest einen Übertragungselement und den Schubelementen eine kraftschlüssige Fixierung bewirkt.

Mit anderen Worten können die Exzenter beispielsweise mit einem nichtrotationssymmetrischen Hohlprofil der Schubelemente zusammenwirken, so dass bei einer Drehung der innenliegenden Exzenter ein Reibschluss und/oder Kraftschluss mit den Schubelementen erzeugt wird. Auf diese Weise können die Schubelemente relativ zueinander in ihrer gegebenen translatorischen Position fixiert werden.

Die Exzenter können als nachgiebige/elastische Exzenter gestaltet sein, die im Klemmzustand mit einer Vorspannkraft auf die Schubelemente einwirken. Es ist jedoch auch vorstellbar, starre Exzenter zu nutzen, deren Klemmwirkung primär auf Reibung beruht.

Gemäß einer weiteren beispielhaften Ausgestaltung weisen die Schubelemente an ihrer Innenseite Anschläge für die Klemmelemente auf, insbesondere für Exzenter der Klemmelemente, wobei die Anschläge eine Endlage für die Rotation der Übertragungselemente im Klemmzustand der Klemmelemente definieren. Auf diese Weise kann ein "Überdrehen" der Klemmelemente vermieden werden. Vorzugsweise gibt es für die Klemmelemente einen definierten Anschlag für den Freigabezustand und einen definierten Anschlag für den Klemmzustand. Auf diese Weise ist die Bedienung eindeutig.

Gemäß einer weiteren beispielhaften Ausgestaltung weisen die Schubelemente ferner an ihrer Innenseite weitere Anschläge für die Klemmelemente auf, wobei die weiteren Anschläge eine Endlage für die Rotation der Übertragungselemente im Freigabezustand der Klemmelemente definieren.

Gemäß einer weiteren beispielhaften Ausgestaltung weist die Haltevorrichtung Folgendes auf:
- genau drei teleskopierbare Schubelemente, umfassend ein proximales Schubelement, ein mittleres Schubelement und ein distales Schubelement, die ein Schubgelenk bilden, wobei die drei Schubelemente drehfest miteinander gekoppelt sind,
- genau drei teleskopierbare Übertragungselemente, umfassend ein proximales Übertragungselement, ein mittleres Übertragungselement und ein distales Übertragungselement, die innerhalb der Schubelemente angeordnet sind,
- ein Drehgelenk, das das erste Gelenk bei der proximalen Basis bildet,
- ein Kugelgelenk, das das zweite Gelenk bei dem distalen Instrumentenhalter bildet,
- ein erstes Klemmelement, das dem Drehgelenk zugeordnet ist und zwischen dem proximalen Übertragungselement und dem Drehgelenk angeordnet ist,
- ein zweites Klemmelement, das zwischen mittleren Übertragungselement und dem proximalen Schubelement angeordnet ist,
- ein drittes Klemmelement, das zwischen dem distalen Übertragungselement und dem mittleren Schubelement angeordnet ist,
- ein viertes Klemmelement, das dem Kugelgelenk zugeordnet ist und zwischen dem distalen Übertragungselement und dem Kugelgelenk angeordnet ist,
- wobei die vier Klemmelemente im Klemmzustand das Drehgelenk, das Schubgelenk und das Kugelgelenk blockieren,
- wobei die vier Klemmelemente jeweils gemeinsam aktivierbar und deaktivierbar sind, und
- wobei die vier Klemmelemente über einen gemeinsamen Antrieb steuerbar sind, um das Drehgelenk, das Schubgelenk und das Kugelgelenk simultan zu blockieren oder freizugeben.
- Aktivieren umfasst ein Sperren/Blockieren. Deaktivieren umfasst ein Lösen bzw. eine Freigabe.

Gemäß einem weiteren Aspekt bezieht sich die vorliegende Offenbarung auf ein medizinisches System, insbesondere endoskopisches System, umfassend eine Haltevorrichtung gemäß einer der hierin beschriebenen Ausgestaltungen sowie ein medizinisches Instrument, das am distalen Instrumentenhalter aufgenommen ist, wobei das medizinische Instrument den distalen Instrumentenhalter durchragt, und wobei das medizinische Instrument entlang einer Instrumentenachse beweglich ist, wenn ein dem zweiten Gelenk zugeordnetes Klemmelement im Freigabezustand ist.

Bei der Instrumentenachse handelt es sich beispielsweise um eine Längsachse durch einen Instrumentenschaft. In einer beispielhaften Ausgestaltung ist das medizinische Instrument um die Längsachse des Instrumentenschafts rotierbar oder entlang der Längsachse verfahrbar, wenn das dem zweiten Gelenk zugeordnete Klemmelement im Freigabezustand ist.

Gemäß einem weiteren Aspekt bezieht sich die vorliegende Offenbarung auf eine Verwendung einer Haltevorrichtung gemäß einer der hierin beschriebenen Ausgestaltungen zur Positionierung eines medizinischen Instruments, insbesondere eines endoskopischen Instruments.

Gemäß einem weiteren Aspekt bezieht sich die vorliegende Offenbarung auf ein Verfahren zur Positionierung eines medizinischen Instruments, insbesondere eines endoskopischen Instruments, mit den folgenden Schritten:
- Bereitstellung einer Haltevorrichtung gemäß einer der hierin beschriebenen Ausgestaltungen,
- Befestigung eines medizinischen Instruments am distalen Instrumentenhalter,
- Positionieren der Haltevorrichtung mit dem Instrument im Freigabezustand der zumindest zwei Klemmelemente, und
- Blockieren der Haltevorrichtung im Klemmzustand der zumindest zwei Klemmelemente.

In einer beispielhaften Ausgestaltung des Verfahrens umfasst der Schritt der Bereitstellung der Haltevorrichtung ferner die Befestigung der Haltevorrichtung, insbesondere der Basis der Haltevorrichtung, an einem Gestell, beispielsweise einer Patientenauflage.

In einer beispielhaften Ausgestaltung ist das Verfahren für andere Zwecke als Verfahren zur chirurgischen oder therapeutischen Behandlung des menschlichen oder tierischen Körpers und Diagnostizierverfahren, die am menschlichen oder tierischen Körper vorgenommen werden, beschränkt. In ähnlicher Weise ist in einer beispielhaften Ausgestaltung die Verwendung auf andere Zwecke als Verfahren zur chirurgischen oder therapeutischen Behandlung des menschlichen oder tierischen Körpers und Diagnostizierverfahren, die am menschlichen oder tierischen Körper vorgenommen werden, beschränkt.

Beispielhaft kann es sich um die Positionierung eines Beobachtungsinstruments handeln, welches den Körper von außerhalb, insbesondere ohne weitere Interaktion mit dem Körper, beobachtet.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Offenbarung zu verlassen.

Weitere Merkmale und Vorteile der Erfindung ergeben sich aus der nachfolgenden Beschreibung und Erläuterung mehrerer beispielhafter Ausführungsbeispiele unter Bezugnahme auf die Zeichnungen. Es zeigen:
- Fig. 1:: eine perspektivische Ansicht eines medizinischen Systems mit einer Haltevorrichtung;
- Fig. 2:: eine perspektivische Ansicht einer beispielhaften Ausführungsform einer Haltevorrichtung;
- Fig. 3:: eine explodierte perspektivische Ansicht der Haltevorrichtung gemäß Fig. 2;
- Fig. 4:: eine gebrochene Seitenansicht der Haltevorrichtung gemäß Fig. 2;
- Fig. 5:: eine gebrochene Schnittansicht der Haltevorrichtung gemäß Fig. 4 entlang der Linie V-V in Fig. 4;
- Fig. 6-9:: frontale Schnittansichten der Haltevorrichtung gemäß Fig. 5 entlang der Linien VI-VI (Fig. 6), VII-VII (Fig. 7), VIII-VIII (Fig. 8) und IX-IX (Fig. 9) in Fig. 5;
- Fig. 10:: eine weitere gebrochene Schnittansicht auf Basis der Darstellung gemäß Fig. 5, wobei aus Veranschaulichungsgründen Elemente ausgeblendet sind;
- Fig. 11:: eine weitere gebrochene Schnittansicht auf Basis der Darstellung gemäß Fig. 5, wobei aus Veranschaulichungsgründen Elemente ausgeblendet sind;
- Fig. 12:: eine schematische geschnittene frontale Ansicht zur Veranschaulichung einer beispielhaften Ausgestaltung eines Klemmelements, in einem Freigabezustand;
- Fig. 13:: eine weitere Ansicht zur Veranschaulichung des Klemmelements gemäß Fig. 12, zur Veranschaulichung eines Klemmzustands; und
- Fig. 14:: ein Flussdiagramm zur Veranschaulichung einer beispielhaften Ausgestaltung eines Verfahrens zur Positionierung eines medizinischen Instruments.

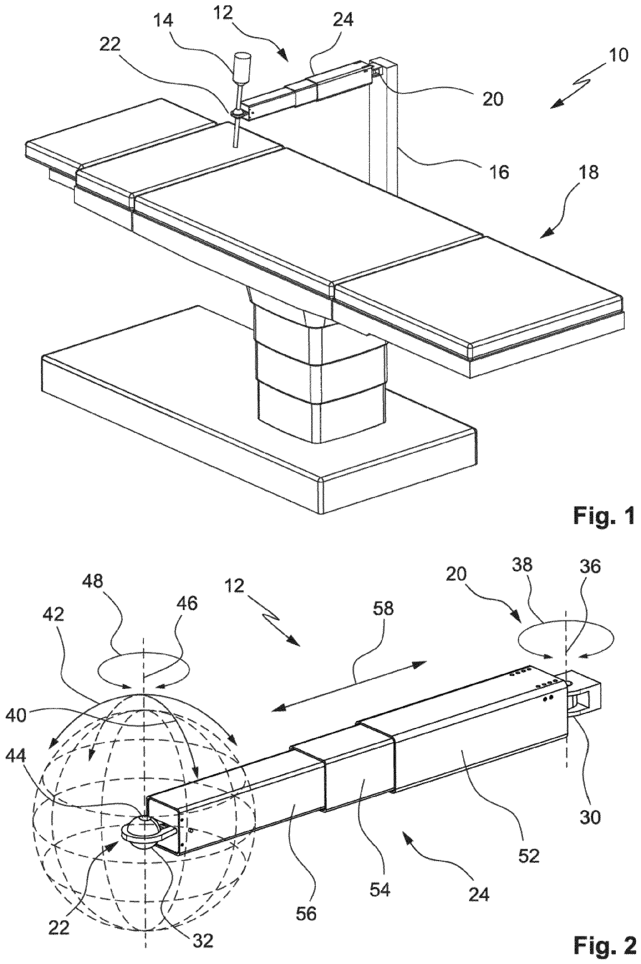

Fig. 1 veranschaulicht anhand einer perspektivischen Darstellung eine Ausführungsform eines mit 10 bezeichneten medizinisches Systems. Das medizinische System 10 weist eine Haltevorrichtung 12 auf, die ein medizinisches Instrument 14 trägt. Die Haltevorrichtung 12 ist über ein Gestell 16 bei einer Patientenauflage 18 angeordnet. Die Haltevorrichtung 12 dient im Ausführungsbeispiel dazu, das Instrument 14 relativ zur Patientenauflage 18 zu positionieren und auszurichten.

Bei dem medizinischen Instrument 14 handelt es sich beispielsweise um ein endoskopisches Instrument, das dazu ausgebildet ist, in den Körper eines Patienten eingeführt zu werden. In anderen Ausgestaltungen handelt es sich bei dem medizinischen Instrument 14 um ein Instrument, das während einer medizinischen Prozedur außerhalb des Körpers eines Patienten platziert ist. Mit anderen Worten kann es sich bei dem medizinischen Instrument 14 beispielsweise um ein endoskopisches Beobachtungsinstrument oder um ein Exoskop handeln. Ein Exoskop ist ein Beobachtungselement zur Beobachtung einer Objektebene von außerhalb des Körpers, also mit einem Arbeitsabstand, der üblicherweise größer als der Arbeitsabstand endoskopischer Beobachtungsinstrumente ist.

Die Haltevorrichtung 12 mit dem daran aufgenommenen Instrument 14 kann in dem in Fig. 1 gezeigten Ausführungsbeispiel in einer Ebene parallel zur Patientenauflage 18 positioniert werden. Zu diesem Zweck weist die Haltevorrichtung 12 eine proximale Basis 20 auf, die am Gestell 16 befestigt ist. Am gegenüberliegenden Ende der Haltevorrichtung 12 ist ein distaler Instrumentenhalter 22 vorgesehen, der das Instrument 14 trägt. Mit anderen Worten erstreckt sich die Haltevorrichtung 12 zwischen einem proximalen Ende und einem distalen Ende. Die Haltevorrichtung 12 umfasst ferner ein Schubgelenk 24, das zwischen der proximalen Basis 20 und dem distalen Instrumentenhalter 22 angeordnet ist.

Mit ergänzender Bezugnahme auf Fig. 2 werden Bewegungsfreiheitsgrade der Haltevorrichtung 12 veranschaulicht. Die proximale Basis 20 weist ein erstes Gelenk (proximales Gelenk) 30 auf, das eine Schwenkachse 36 definiert. Mit anderen Worten kann das erste Gelenk 30 auch als Schwenkgelenk bezeichnet werden. Ein mit 38 bezeichneter gekrümmter Doppelpfeil veranschaulicht eine mögliche Rotation/Schwenkbewegung der Haltevorrichtung 12 relativ zum Gestell 16 (vergleiche Fig. 1). Auf diese Weise kann die Haltevorrichtung 12 mit dem Instrument 14 ähnlich einem Auslegerarm um die Schwenkachse 36 verschwenkt werden.

Der distale Instrumentenhalter 22 weist ein zweites Gelenk (distales Gelenk) 32 auf, das Schwenkbewegungen des Instruments 14 ermöglicht. Im Ausführungsbeispiel gemäß Fig. 2 ist das zweite Gelenk 32 als Kugelgelenk gestaltet. Gekrümmte Doppelpfeile 40, 42 veranschaulichen denkbare Schwenkbewegungen des Instruments 14, die durch das zweite Gelenk 32 ermöglicht werden. Das zweite Gelenk 32 umfasst eine Instrumentenaufnahme 44, die etwa als Aufnahmebohrung für das Instrument 14 gestaltet ist. Im Ausführungsbeispiel gemäß Fig. 2 definiert die Instrumentenaufnahme 44 eine Instrumentenachse 46 für das aufgenommene Instrument 14 (vergleiche Fig. 1). In einer beispielhaften Ausgestaltung kann das Instrument 14 mit einem Instrumentenschaft in die Instrumentenaufnahme 44 eingeführt und dort gehalten werden. In einer beispielhaften Ausgestaltung ist das Instrument 14 in der Instrumentenaufnahme 44 um die Instrumentenachse 46 drehbar, vergleiche die Rotationsbewegung 48, die anhand eines gekrümmten Doppelpfeils veranschaulicht wird. Auch translatorische Bewegungen entlang der Instrumentenachse 46 sind denkbar.

Zwischen dem ersten Gelenk 30 und dem zweiten Gelenk 32 erstreckt sich das Schubgelenk 24. Das Schubgelenk 24 umfasst im Ausführungsbeispiel drei Schubelemente 52, 54, 56. Es versteht sich, dass das Schubgelenk 24 grundsätzlich zwei oder mehr Schubelemente umfassen kann, beispielsweise zwei, drei oder vier Schubelemente. Das Schubgelenk 24 erlaubt eine Translationsbewegung, vergleiche den Doppelpfeil 58 in Fig. 2. Mit anderen Worten erlaubt das Schubgelenk 24 eine Ausfahrbarbewegung oder Einfahrbewegung der Schubelemente 52, 54, 56.

Insgesamt weist die Haltevorrichtung 12 zumindest drei Gelenke auf, nämlich das erste Gelenk 30, das zweite Gelenk 32 und das dazwischenliegende Schubgelenk 24. Das Schubgelenk 24 stellt eine translatorische Bewegung bereit. Das im Ausführungsbeispiel als Drehgelenk gestaltete erste Gelenk 30 erlaubt eine Schwenkbewegung. Das im Ausführungsbeispiel als Kugelgelenk gestaltete zweite Gelenk 32 erlaubt Schwenkbewegungen um mehrere Achsen. Die Gelenke 24, 30 dienen zur Positionierung des Instrumentenhalters 22 in einer Ebene. Der Bereich, in dem eine Positionierung möglich ist, wird durch den möglichen Schwenkwinkel des Drehgelenks 30 und den Hub (Verfahrweg) des Schubgelenks 24 definiert. Die Ebene ist beispielsweise parallel zur Patientenauflage (Fig. 18) orientiert. Das Gelenk 32 erlaubt im Ausführungsbeispiel die Ausrichtung des am Instrumentenhalter 22 gehaltenen Instruments 14. Dies kann Schwenkbewegungen im als Kugelgelenk gestalteten Gelenk 32 umfassen. In beispielhaften Ausgestaltungen kann das Instrument 14 ferner auch translatorisch in der Instrumentenaufnahme 44 und rotatorisch um die Instrumentenachse 46 bewegt werden.

In einer beispielhaften Ausgestaltung erfolgt die Bewegung und Ausrichtung des Instruments 14 über die Haltevorrichtung 12 manuell. Mit anderen Worten kann das medizinische Personal das Instrument 14 händisch platzieren und ausrichten. In dieser Ausgestaltung sind keine eigenen Antriebe für die Bewegungsfreiheitsgrade der Gelenke 24, 30, 32 erforderlich. Es versteht sich, dass grundsätzlich auch Gestaltungen vorstellbar sind, bei denen auch die Positionierung und Ausrichtung des Instruments 14 über Motoren/Aktoren erfolgt. Es hat sich jedoch gezeigt, dass in verschiedenen Anwendungen die händische Ausrichtung absolut ausreichend ist.

Wesentlich ist jedoch die Möglichkeit, die Haltevorrichtung 12 definiert blockieren oder lösen zu können. In einem blockierten oder gesperrten/gehemmten Zustand der Haltevorrichtung 12 ist das Instrument 14 hinreichend fest positioniert und orientiert. Mit anderen Worten ist in diesem Zustand eine weiterführende Bewegung des Instruments 14 und/oder der Haltevorrichtung 12 unterbunden, gegebenenfalls nur mit hohem Kraftaufwand möglich. Wenn nun jedoch das Instrument 14 umpositioniert werden soll oder eine neue Ausrichtung des Instruments 14 gewünscht ist, so muss der gesperrte Zustand (auch bezeichnet als Klemmzustand) aufgehoben werden, damit die Haltevorrichtung 12 mit dem Instrument 14 in den gewünschten Freiheitsgraden bewegt werden kann. Es ist daher vorteilhaft, wenn das Blockieren und Lösen der Haltevorrichtung 12 einfach bewerkstelligt werden kann. Vorzugsweise ist für den Bediener (Operateur) nur eine Aktion erforderlich, um die gesamte Haltevorrichtung 12 zu sperren oder freizugeben.

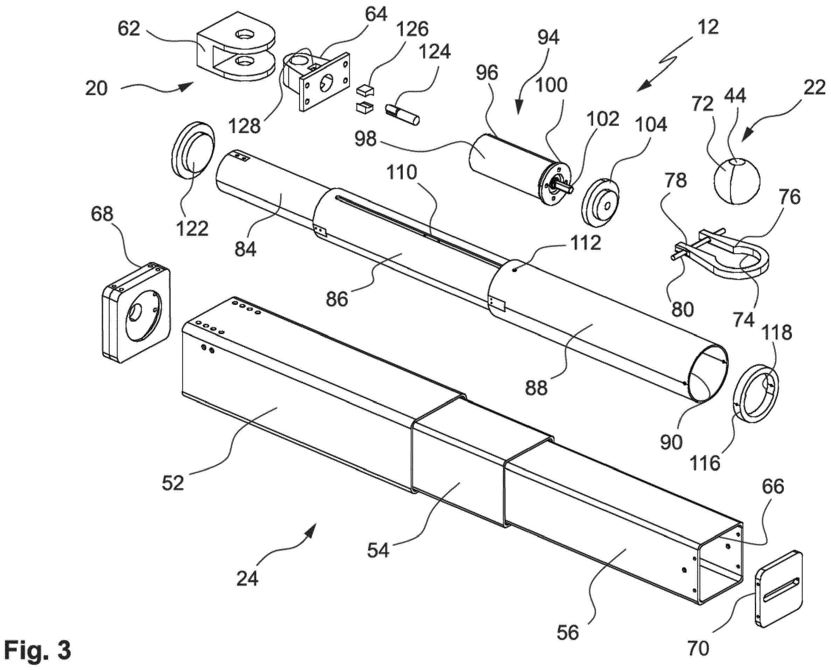

Mit Bezugnahme auf die Figuren 3-12 sowie mit ergänzender Bezugnahme auf die Figuren 1 und 2 werden beispielhafte Ausgestaltungen der Haltevorrichtung 12 näher erläutert. Verschiedene Aspekte der vorliegenden Offenbarung befassen sich mit dem simultanen Blockieren bzw. Lösen der Gelenke 24, 30, 32.

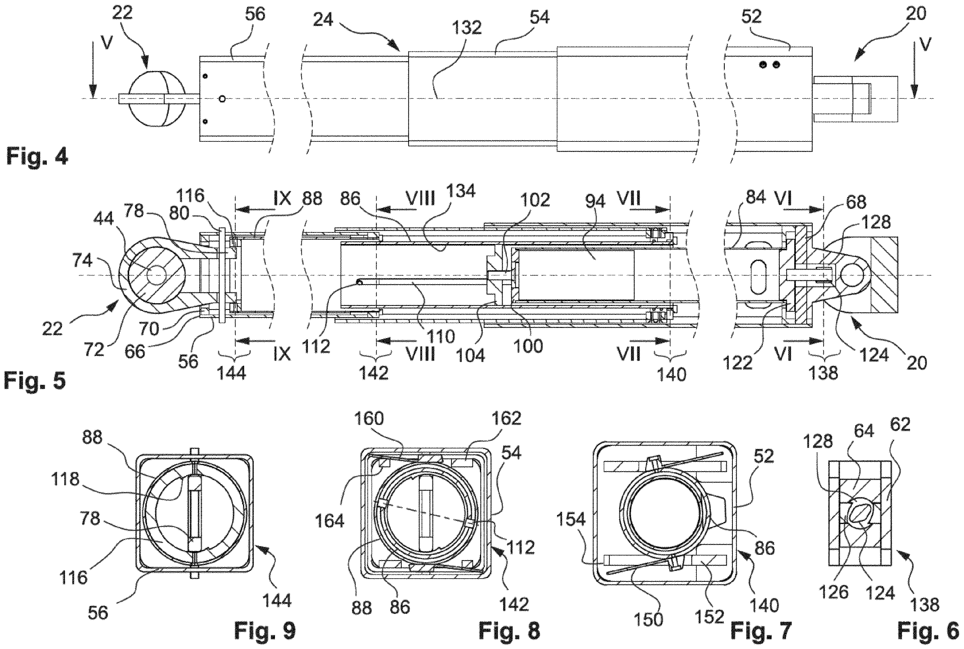

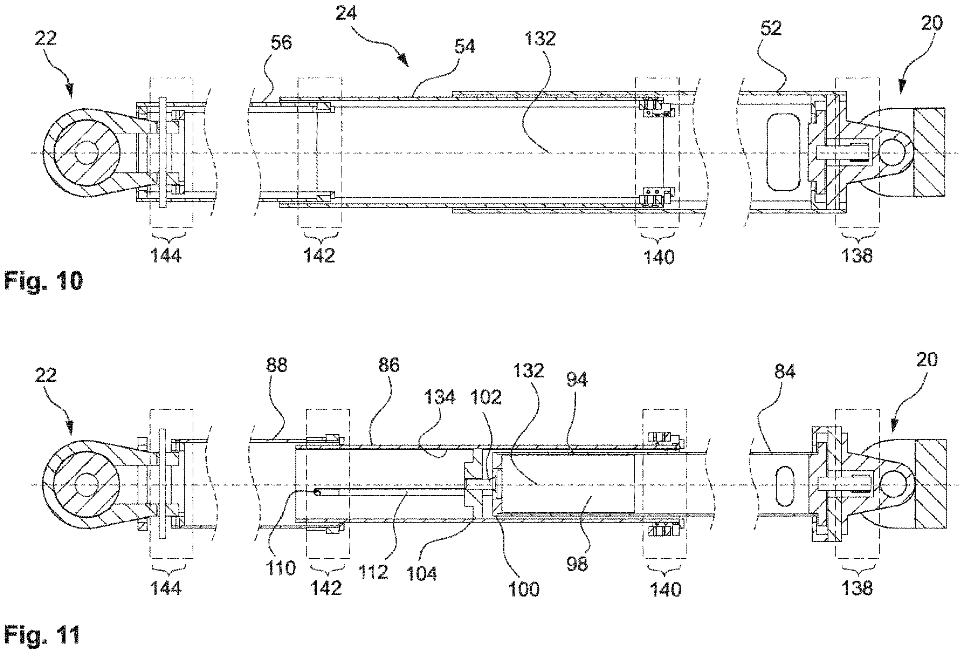

Fig. 3 veranschaulicht anhand einer perspektivischen, explodierten Darstellung eine beispielhafte Detailgestaltung der Haltevorrichtung 12 gemäß Fig. 2. Die Figuren 4-11 ergänzen diese Darstellung. Fig. 4 zeigt eine gebrochene Seitenansicht der Haltevorrichtung 12 gemäß den Figuren 2 und 3. Fig. 5 zeigt eine korrespondierende Schnittansicht, wobei die Schnittebene durch die Pfeile V-V in Fig. 4 angedeutet ist. Aufbauend auf den Pfeilen VI-VI, VII-VII, VIII-VIII sowie IX-IX zeigen die Figuren 6, 7, 8 und 9 frontale Schnittansichten quer zur Längserstreckung der Haltevorrichtung 12. die Figuren 10 und 11 basieren auf Fig. 5, wobei jeweils aus Veranschaulichungsgründen verschiedene Komponenten der Haltevorrichtung 12 ausgeblendet sind.

Die das erste Gelenk 30 bildende Basis 20 weist eine Aufnahme 62 und ein Schwenkstück 64 auf, das an der Aufnahme 62 schwenkbar gelagert ist. Die Aufnahme 62 ist im Ausführungsbeispiel gemäß Fig. 1 am Gestell 16 befestigt. In Fig. 3 wurde aus Gründen der Übersichtlichkeit auf die Darstellung eines Bolzens verzichtet, der die Aufnahme 62 und das Schwenkstück 64 miteinander verbindet.

Die proximale Basis 20 ist mit dem Schubgelenk 24 gekoppelt. Das Schubgelenk 24 ist im Ausführungsbeispiel ersichtlich aus den Schubelementen 52, 54, 56 gebildet. Die Schubelemente 52, 54, 56 weisen jeweils einen Profilkörper 66 mit einem Querschnittsprofil auf. In Fig. 3 handelt es sich jeweils um ein Vierkantprofil bzw. Rechteckprofil. Die Profilkörper 66 der Schubelemente 52, 54, 56 sind aneinander angepasst, so dass das Schubelement 56 innerhalb des Schubelements 54 angeordnet ist, und dass das Schubelement 54 innerhalb des Schubelements 52 angeordnet ist. Dies bezieht sich jeweils auf den Querschnitt des jeweiligen Profilkörpers 66. Auf diese Weise können die Schubelemente 52, 54, 56 ineinander eintauchen bzw. relativ zueinander zwischen einem eingefahrenen und einem ausgefahrenen Zustand translatorisch bewegt werden. Die Schubelemente 52, 54, 56 sind aufgrund ihres Profilkörper 66 drehfest miteinander verbunden.

Das Schwenkstück 64 der proximalen Basis 20 ist mit einer proximalen Kappe 68 verbunden, die wiederum mit dem proximalen Schubelement 52 gekoppelt ist. Auf diese Weise ist das Schubelement 52 des Schubgelenks 24 im gezeigten Ausführungsbeispiel fest mit dem Schwenkstück 64 verbunden. Bei einer Schwenkbewegung zwischen dem Schwenkstück 64 und der Aufnahme 62 wird folglich auch das Schubgelenk 24 verschwenkt. Am distalen Ende des distalen Schubelements 56 ist eine Kappe 70 vorgesehen, die mit dem Schubelement 56 verbunden ist. Auf diese Weise wird im Schubgelenk 24 ein Innenraum definiert, der weitere Komponenten der Haltevorrichtung 12 beherbergt.

Der distalen Instrumentenhalter 22 umfasst eine Führungskugel 72, die in einer Kugelpfanne 74 angeordnet ist. Die Kugelpfanne 74 ist im Ausführungsbeispiel gemäß Fig. 3 als Joch 76 bzw. jochartig gestaltet. Die Kugelpfanne 74 stellt eine Aufnahme für die Führungskugel 72 mit einer sphärisch geformten Fläche bereit, die an die Außenfläche der Führungskugel 72 angepasst ist. An die Kugelpfanne 74 schließen sich zwei Schenkel 78 an. Die beiden Schenkel 78 sind ausgehend von der distal angeordneten Kugelpfanne 74 proximal orientiert.

Die Kugelpfanne 74 und die beiden Schenkel 78 sind im Ausführungsbeispiel nach Art eines Jochs 76 gestaltet. Im montierten Zustand (vergleiche Fig. 2 und Fig. 4) durchragen die Schenkel 78 eine schlitzartige Ausnehmung in der Kappe 70. Im montierten Zustand durchragt ein Stift 80 die Schenkel 78 sowie das distale Ende des Schubelements 56, vergleiche hierzu Fig. 4 und Fig. 5. Der Stift 80 und die Gestaltung des Schlitzes in der Kappe 70 sorgen für eine verdrehsichere Abstützung des distalen Instrumentenhalters 22 am distalen Schubelement 56. Mit anderen Worten kann die jochartig gestaltete Kugelpfanne 74 nicht oder nur unmerklich um eine Längsachse durch die Haltevorrichtung 12 (vergleiche Bezugszeichen 132 in Fig. 4) verschwenkt werden.

Innerhalb des Schubgelenks 24 sind im Ausführungsbeispiel gemäß Fig. 3 Übertragungselemente 84, 86, 88 angeordnet. In einer beispielhaften Ausgestaltung ist die Anzahl der Übertragungselemente 84, 86, 88 an die Anzahl der Schubelemente 52, 54, 56 angepasst. Ein proximales Übertragungselement 84 ist der proximalen Basis 20 zugewandt. Ein distales Übertragungselement 88 ist dem distalen Instrumentenhalter 22 zugewandt. Zwischen den Übertragungselementen 84 und 88 ist das mittlere Übertragungselement 86 angeordnet. Die Übertragungselemente 84, 86, 88 weisen Profilkörper 90 auf, die beispielhaft als Rohrstücke gestaltet sind. Mit anderen Worten haben die Profilkörper 90 einen kreisförmigen bzw. kreisringförmigen Querschnitt. Im Ausführungsbeispiel sind die Übertragungselemente 84, 86, 88 als Hohlzylinder gestaltet. In der Gestaltung gemäß Fig. 3 weist das distale Übertragungselement 88 einen größeren Querschnitt als das mittlere Übertragungselement 86 auf, wobei das mittlere Übertragungselement 86 einen größeren Querschnitt als das proximale Übertragungselement 84 aufweist.

Im Ausführungsbeispiel verjüngen sich die Schubelemente 52, 54, 56 von proximal nach distal. Im Ausführungsbeispiel verjüngen sich die Übertragungselemente 84, 86, 88 von distal nach proximal.

Die Schubelemente 52, 54, 56 des Schubgelenks 24 sind translatorisch verfahrbar aber drehfest miteinander gekoppelt. Die Übertragungselemente 84, 86, 88 sind innerhalb der Schubelemente 52, 54, 56 angeordnet. Die Übertragungselemente 84, 86, 88 sind zumindest zwischen einer ersten Position (einem Freigabezustand zugeordnet) und einer zweiten Position (einem Klemmzustand zugeordnet) rotierbar. Die Übertragungselemente 84, 86, 88 sind absolut (gegenüber der Umgebung bzw. dem Schubgelenk 24) rotierbar, wobei zumindest einige der Übertragungselemente 84, 86, 88 relativ zueinander rotierbar sind. Dies wird nachfolgend näher veranschaulicht.

Das proximale Übertragungselement 84 ist ähnlich wie das proximale Schubelement 52 mit der proximalen Basis 20 gekoppelt. In einer beispielhaften Ausgestaltung gibt es zwischen der Basis 20, dem maximal Schubelement 52 und dem proximalen Übertragungselement 84 keine translatorische Bewegung. Das distale Übertragungselement 88 ist in einer beispielhaften Ausgestaltung zur translatorischen Bewegungsmitnahme mit dem distalen Schubelement 56 gekoppelt. Wenn also das Schubgelenk 24 ausgezogen wird, werden auch die Übertragungselemente 84, 86, 88 auseinandergezogen (expandiert). Im gezeigten Ausführungsbeispiel ist das Übertragungselement 86 relativ zum Übertragungselement 84 und relativ zum Übertragungselement 88 translatorisch verfahrbar. Die Ausfahrbewegung und Einfahrbewegung der Übertragungselemente 84, 86, 88 ist an die Ausfahrbewegung und Einfahrbewegung der Schubelemente 52, 54, 56 angepasst.

In beispielhaften Ausführungsformen ist ein Antrieb 94 vorgesehen, um die Haltevorrichtung 12 wahlweise zu blockieren oder freizugeben. Auf diese Weise kann ein Instrument 14, das am Instrumentenhalter 22 befestigt ist, positioniert und in einer gewünschten Ausrichtung orientiert werden. Fig. 3 veranschaulicht, dass der Antrieb 94 einen Motor 96 umfasst, der ein beispielsweise patronenartig oder kartuschenartig gestaltetes Gehäuse 98 aufweist. Der Motor 96 ist beispielhaft als Elektromotor gestaltet. Der Motor 96 ist mit einer nicht gezeigten Steuereinrichtung gekoppelt, die zumindest einen Schalter zum Aktivieren und/oder Deaktivieren des Motors umfasst. Die Aktivierung kann bedarfsweise eine Aktivierung in einer ersten Drehrichtung oder eine Aktivierung in einer zweiten Drehrichtung umfassen. Mit anderen Worten weisen die Steuereinrichtung bzw. der zumindest eine Schalter in einer beispielhaften Ausgestaltung drei Schaltstellungen für den Antrieb auf.

Am Gehäuse 98 ist im Ausführungsbeispiel ein Lagerstück 100 ausgebildet, das den Antrieb 94 am proximalen Übertragungselement 84 festlegt, vergleiche auch Fig. 5 sowie Fig. 11. Mit anderen Worten stützt sich der Antrieb 94 gehäuseseitig am Übertragungselement 84 ab. Der Antrieb 94 umfasst ferner eine Antriebswelle 102, die im Ausführungsbeispiel mit einem Mitnehmer 104 gekoppelt ist. Der Mitnehmer 104 überträgt eine rotatorische Antriebsbewegung des Antriebs 94 auf das mittlere Übertragungselement 86, vergleiche Fig. 5 und Fig. 11. Wenn der Antrieb 94 aktiviert ist, werden auf diese Weise die Übertragungselemente 84 und 86 gegensinnig verdreht. Der Antrieb 94 wirkt über die Antriebswelle 102 und den Mitnehmer 104 mit einem Antriebsmoment auf das Übertragungselement 86. Gleichsam wirkt ein dabei entstehendes Reaktionsmoment über das Lagerstück 100 auf das proximale Übertragungselement 84. Auf diese Weise ergibt sich eine kompakte, integrale Gestaltung, weil der Antrieb 94 nicht zwingend gestellseitig gelagert sein muss.

Die beiden Übertragungselemente 86, 88 sind drehfest miteinander gekoppelt und translatorisch relativ zueinander verfahrbar. Mit anderen Worten bilden die beiden Übertragungselemente 86, 88 gemeinsam ein (weiteres) Schubgelenk, das jedoch insgesamt (absolut) durch den Antrieb 94 rotierbar ist. Damit rotieren die Übertragungselemente 86, 88 bei aktiviertem Antrieb 94 in eine erste Richtung, wohingegen das Übertragungselement 84 in eine gegensinnige zweite Richtung rotiert. Diese Gestaltung erlaubt den Verzicht auf eine separate gestellfeste Lagerung des Antriebs 94. Gleichwohl sind auch Haltevorrichtungen mit einheitlich rotierenden Übertragungselementen vorstellbar, welche dann beispielhaft nicht zylindrisch gestaltet sein müssen.

Das proximale Übertragungselement 84 und das mittlere Übertragungselement 86 sind mittelbar über den Antrieb 94 bzw. über dessen Lagerstück 100 sowie den Mitnehmer 104 miteinander gekoppelt und relativ zueinander translatorisch verfahrbar und rotierbar. Das mittlere Übertragungselement 86 und das distale Übertragungselement 88 sind über zumindest eine Führung 110 sowie zumindest ein darin angeordnetes Führungselement 112 translatorisch und drehfest miteinander gekoppelt. Bei der Führung 110 handelt sich beispielsweise um ein Langloch bzw. einen Führungsschlitz. Demgemäß ist das Führungselement 112 beispielsweise als Führungsstift oder Bolzen gestaltet, der in der Führung 110 angeordnet ist. Gemeinsam sorgen die Führung 110 und das Führungselement 112 für die translatorische Verschiebbarkeit und die drehfeste Verbindung zwischen dem Übertragungselement 86 und dem Übertragungselement 88. Auf diese Weise wird eine Rotationsbewegung, die über den Mitnehmer 104 in das Übertragungselement 86 eingeleitet wird, auch auf das Übertragungselement 88 übertragen.

Das distale Übertragungselement 88 ist an seinem distalen Ende mit einem Klemmring 116 gekoppelt, der einen Exzenter 118 bildet bzw. Exzenterkonturen aufweist. Der Klemmring 116 ist drehfest mit dem Übertragungselement 88 gekoppelt und wird gemeinsam mit diesem rotiert. Der Klemmring 116 wirkt bedarfsweise mit dem nach innen gerichteten Exzenter 118 auf die Schenkel 78 der jochartig gestalteten Kugelpfanne 74 ein, um diese zusammenzudrücken, vergleiche auch Fig. 5 und Fig. 9.

Auf diese Weise können die Schenkel 78 des Joch 76 bedarfsweise verspannt werden, um die Führungskugel 72 in der Kugelpfanne 74 zu fixieren. Auf diese Weise kann das zweite Gelenk (Kugelgelenk) 32 bedarfsweise gesperrt oder blockiert werden. In einer beispielhaften Ausgestaltung ist die Führungskugel 72 im diesem Zustand (Klemmzustand) fest mit der Kugelpfanne 74 verbunden, so dass keine Schwenkbewegungen (vergleiche die Pfeile 40, 42 in Fig. 2) möglich sind. In einer weiteren beispielhaften Ausgestaltung ist zusätzlich auf die Instrumentenaufnahme 44 vorgespannt, so dass ein aufgenommenes Instrument 14 nicht mehr um die Instrumentenachse 46 rotiert werden kann, vergleiche ebenso Fig. 2. Im gelösten/entsperrten Zustand (Freigabezustand) ist der Klemmring 116 derart verdreht, dass der Exzenter 118 die Schenkel 78 nicht mehr zusammendrückt. In diesem Zustand kann die Führungskugel 72 in der Kugelpfanne 74 verschwenkt werden. Bedarfsweise ist auch eine Rotation des Instruments 14 um die Instrumentenachse 46 in der Instrumentenaufnahme 44 und gegebenenfalls ein Verschieben entlang der Instrumentenachse 46 ermöglicht.

Das proximale Übertragungselement 84 ist an seinem proximalen Ende mit einem Verbindungsstück 122 gekoppelt, das im Ausführungsbeispiel scheibenartig oder kappenartig gestaltet ist. Das Verbindungsstück 122 ist drehfest mit dem Übertragungselement 84 gekoppelt und wird gemeinsam mit diesem rotiert. Das Verbindungsstück 122 trägt einen Exzenter 124, der im Ausführungsbeispiel als proximale Verlängerung des Verbindungsstücks 122 gestaltet ist. Der Exzenter 124 ragt im Ausführungsbeispiel in eine Ausnehmung 128 im Schwenkstück 64 der proximalen Basis 62 hinein, vergleiche auch Fig. 5 und Fig. 6.

In der Ausnehmung 128 des Schwenkstücks 64 ist zumindest ein Stempel 126 angeordnet. Der Exzenter 124 wirkt bedarfsweise auf den zumindest einen Stempel 126 ein und drängt diesen radial nach außen, um das Schwenkstück 64 relativ zur Aufnahme 62 festzulegen (zu blockieren bzw. zu sperren). Die Drehbewegung des Exzenters 124 wird durch den Antrieb 94 eingeleitet. Auf diese Weise kann das erste Gelenk (Drehgelenk) 30 blockiert werden (Klemmzustand). Im gelösten/entsperrten Zustand (Freigabezustand) ist der Exzenter 124 derart verdreht, dass der zumindest eine Stempel 126 in der Ausnehmung 128 nicht mehr gegen Aufnahme 62 vorgespannt ist. Damit ist das erste Gelenk 30 freigegeben.

Im Klemmzustand ist es jedoch auch erforderlich, translatorische Bewegung des Schubgelenks 24 zu sperren bzw. zu hemmen. Mit Bezugnahme auf die Figuren 4-11 werden einschlägige Gestaltungen veranschaulicht. Die translatorische Bewegung der Schubelemente 52, 54, 56 des Schubgelenks 24 korrespondiert im gezeigten Ausführungsbeispiel mit einer translatorischen Bewegung der Übertragungselemente 84, 86, 88. Die Übertragungselemente 86, 88 sind über zumindest eine Führung 110 und ein Führungselement 112, das in die Führung 110 eingreift, translatorisch und drehfest miteinander gekoppelt, vergleiche auch Fig. 4. Zusätzlich veranschaulicht Fig. 4 eine mit 134 bezeichnete Führung am Übertragungselements 86 für den Mitnehmer 104 des Antriebs 94. Der Mitnehmer 104 weist beispielsweise einen Vorsprung (nicht detailliert dargestellt) auf, der in die Führung 134 eingreift. Auf diese Weise ist eine translatorische Bewegung zwischen dem Antrieb 94 bzw. dessen Mitnehmer 104 und dem Übertragungselement 86 ermöglicht, mithin eine translatorische Relativbewegung zwischen den Übertragungselementen 84 und 86.

Wenn der Antrieb 94 aktiviert wird, dreht sich der Mitnehmer 104 relativ zum Lagerstück 100. Das Lagerstück 100 ist mit dem Übertragungselement 84 gekoppelt. Der Mitnehmer 104 ist mit dem Übertragungselement 86 gekoppelt. Auf diese Weise ergibt sich eine Relativrotation zwischen den Übertragungselement 84, 86. Das Übertragungselement 88 rotiert gemeinsam mit dem Übertragungselement 86. Je nach Drehrichtung des Antriebs 94 drehen also beispielsweise die Übertragungselemente 86, 88 im Uhrzeigersinn, wogegen das Übertragungselemente 84 gleichzeitig im Gegenuhrzeigersinn rotiert. Eine umgekehrte Zuordnung ist denkbar.

Diese Drehbewegungen werden zum einen zum bedarfsweisen Blockieren des ersten Gelenks 30 und des zweiten Gelenks 32 (Fig. 2) genutzt. Jedoch lässt sich die Drehbewegung ebenso nutzen, um das Schubgelenk 24 bedarfsweise zu sperren. In Fig. 4 sowie ergänzend in Fig. 11 sind schematisch sogenannte Klemmelemente 138, 140, 142, 144 angedeutet, die die Gelenke 24, 30, 32 bedarfsweise klemmen oder freigeben. Im Klemmzustand sind die Gelenke 24, 30, 32 blockiert, zumindest hinreichend gehemmt. Im Freigabezustand sind die Gelenke 24, 30, 32 gelöst bzw. zumindest hinreichend beweglich, um das Instrument 14 positionieren zu können. Fig. 6 veranschaulicht einen Schnitt durch das Klemmelement 138. Fig. 7 veranschaulicht einen Schnitt durch das Klemmelement 140. Fig. 8 veranschaulicht einen Schnitt durch das Klemmelement 142. Fig. 9 veranschaulicht einen Schnitt durch das Klemmelement 144.

Das proximale Klemmelement 138 umfasst den Exzenter 124, der mit dem zumindest einen Stempel 126 zusammenwirkt, um das Schwenkstück 64 und die Aufnahme 62 gegeneinander zu verspannen. Die Antriebskraft bzw. das Antriebsmoment wird vom Antrieb 94 über dessen Lagerstück 100 auf das proximale Übertragungselement 84, vom Übertragungselement 84 auf das Verbindungsstück 122 und vom Verbindungsstück 122 auf den als Fortsatz gestalteten Exzenter 124 übertragen, um das als Drehgelenk gestaltete erste Gelenk 30 zu sperren. Fig. 6 zeigt eine Freigabestellung des proximalen Klemmelements 138.

Das distale Klemmelement 144 umfasst den mit dem Exzenter 118 versehenen Klemmring 116, der auf Schenkel 78 der Kugelpfanne 74 einwirkt. Auf diese Weise können die Kugelpfanne 74 und die Führungskugel 72 gegeneinander verspannt werden. Die Antriebskraft bzw. das Antriebsmoment wird vom Antrieb 94 über dessen Mitnehmer 104 auf das mittlere Übertragungselement 86, vom mittleren Übertragungselement 86 auf das distale Übertragungselement 88 und von dort über den Klemmring 116 und dessen Exzenter 118 auf den oder die Schenkel 78 übertragen, um das als Kugelgelenk gestaltete zweite Gelenk 32 zu sperren. Fig. 9 zeigt eine Freigabestellung des distalen Klemmelements 144.

Zwischen dem proximalen Klemmelement 138 und dem distalen Klemmelement 144 ist zumindest ein weiteres Klemmelement 140, 142 vorgesehen, um das Schubgelenk 24 zu sperren. Im Ausführungsbeispiel sind zwei Klemmelemente 140, 142 vorgesehen, die zwischen dem proximalen Klemmelement 138 und dem distalen Klemmelement 144 angeordnet sind.

Fig. 5 und Fig. 11 veranschaulichen, dass die Klemmelemente 140, 142 über das mittlere Übertragungselement 86 bzw. das distale Übertragungselement 88 betätigt werden. Der Antrieb 94 ist über den Mitnehmer 104 mit dem mittleren Übertragungselement 86 sowie über die Führung 110 und das Führungselement 112 mit dem distalen Übertragungselement 88 gekoppelt.

Das Klemmelement 140 ist beim proximalen Ende des mittleren Übertragungselements 86 ausgebildet. Beim proximalen Endbereich des mittleren Übertragungselements 86 ist ein als Exzenterfeder gestalteter Exzenter 150 ausgebildet. Fig. 7 veranschaulicht, dass beispielsweise zwei derartige Exzenterfedern 150 vorgesehen sein können, die um 180° versetzt angeordnet sind. Bei einer Rotation (in Fig. 7 im Gegenuhrzeigersinn) des mittleren Übertragungselements 86 werden die Exzenterfedern 150 aus einer in Fig. 7 gezeigten Freigabestellung (Exzenterfedern 150 kontaktieren das proximale Schubelement 52 nicht) in eine Klemmstellung überführt, in der die Exzenterfedern 150 das proximale Schubelement 52 kontaktieren. Den Exzenterfedern 150 sind ferner Anschläge 152, 154 zugeordnet, die Endbereiche der Bewegung zwischen der Klemmstellung (Anschlag 152) und der Freigabestellung (Anschlag 154) definieren. Die Anschläge 152, 154 sind beispielsweise beim mittleren Schubelement 54 vorgesehen.

Die Exzenterfedern 150 sind ferner dem proximalen Ende des mittleren Schubelements 54 benachbart, vergleiche Fig. 5. Wenn die Exzenterfedern 150 nahe beim proximalen Ende des Schubelements 54 auf das proximale Schubelement 52 einwirken, ergibt sich ein großer nutzbarer Verfahrweg bei der translatorischen Bewegung. Die Exzenterfedern 150 bzw. das Klemmelement 140 sind derart angeordnet, dass eine gemeinsame translatorische Bewegung mit dem mittleren Schubelement 54 und dem mittleren Kraftübertragungselement 86 erfolgt, wenn das Schubgelenk 24 ausgefahren oder eingefahren wird.

Das Klemmelement 142 ist beim proximalen Ende des distalen Übertragungselements 88 ausgebildet. Beim proximalen Endbereich des distalen Übertragungselements 88 ist ein als Exzenterfeder gestalteter Exzenter 160 ausgebildet. Fig. 8 veranschaulicht, dass beispielsweise zwei derartige Exzenterfedern 160 vorgesehen sein können, die um 180° versetzt angeordnet sind. Bei einer Rotation (in Fig. 8 im Uhrzeigersinn) des mittleren Übertragungselements 86 und folglich des distalen Übertragungselements 88 werden die Exzenterfedern 160 aus einer in Fig. 8 gezeigten Freigabestellung (Exzenterfedern 160 kontaktieren das mittlere Schubelement 54 nicht oder nur mit geringer Kraft) in eine Klemmstellung überführt, in der die Exzenterfedern 160 das mittlere Schubelement 54 mit einer gewissen Kraft/Vorspannung kontaktieren. Den Exzenterfedern 160 sind ferner Anschläge 162, 164 zugeordnet, die Endbereiche der Bewegung zwischen der Klemmstellung (Anschlag 162) und der Freigabestellung (Anschlag 164) definieren. Die Anschläge 162, 164 sind beispielsweise am distalen Schubelement 56 vorgesehen.

Die Exzenterfedern 160 sind ferner dem proximalen Ende des distalen Schubelements 56 benachbart, vergleiche Fig. 5. Wenn die Exzenterfedern 160 nahe beim proximalen Ende des distalen Schubelements 56 auf das mittlere Schubelement 54 einwirken, ergibt sich ein großer nutzbarer Verfahrweg bei der translatorischen Bewegung. Die Exzenterfedern 160 bzw. das Klemmelement 142 sind derart angeordnet, dass eine gemeinsame translatorische Bewegung mit dem distalen Schubelement 56 und dem distalen Kraftübertragungselement 88 erfolgt, wenn das Schubgelenk 24 ausgefahren oder eingefahren wird.

Die Klemmelemente 138, 140, 142, 144 werden simultan über den Antrieb 94 gesteuert. Der Antrieb 94 kann in einer ersten Drehrichtung betrieben werden, um die Klemmelemente 138, 140, 142, 144 in den Klemmzustand zu bringen. Der Antrieb 94 kann in einer entgegengesetzten, zweiten Drehrichtung betrieben werden, um die Klemmelemente 138, 140, 142, 144 in den Freigabezustand zu bringen.

Es versteht sich, dass die Klemmelemente 138, 140, 142, 144, zumindest einige der Klemmelemente 138, 140, 142, 144, auch anderweitig gestaltet sein können. Im Ausführungsbeispiel ergibt sich mit den Klemmelemente 138, 140, 142, 144 der Vorteil, dass eine Mehrzahl von Bewegungsfreiheitsgraden der Haltevorrichtung 12 mit nur einem Antrieb bzw. nur einem Schritt simultan gesperrt oder freigegeben werden kann.

Auf Basis der Darstellung gemäß Fig. 5 veranschaulichen die Figuren 10 und 11 weitere Merkmale der Haltevorrichtung 12. Fig. 10 zeigt eine gebrochene Schnittansicht, bei der bewusst auf die Darstellung der Übertragungselemente 84, 86, 88 und des Antriebs 94 verzichtet wurde. Fig. 11 zeigt eine gebrochene Schnittansicht, bei der bewusst auf die Darstellung der Schubelemente 52, 54, 56 des Schubgelenks 24 verzichtet wurde. Die Klemmelemente 138, 140, 142, 144 sind in den Figuren 10 und 11 durch gestrichelte Kästchen angedeutet, um zu veranschaulichen, dass auch anderweitige Gestaltungen vorstellbar sind, um die beteiligten Elemente bedarfsweise zu sperren oder zu lösen.

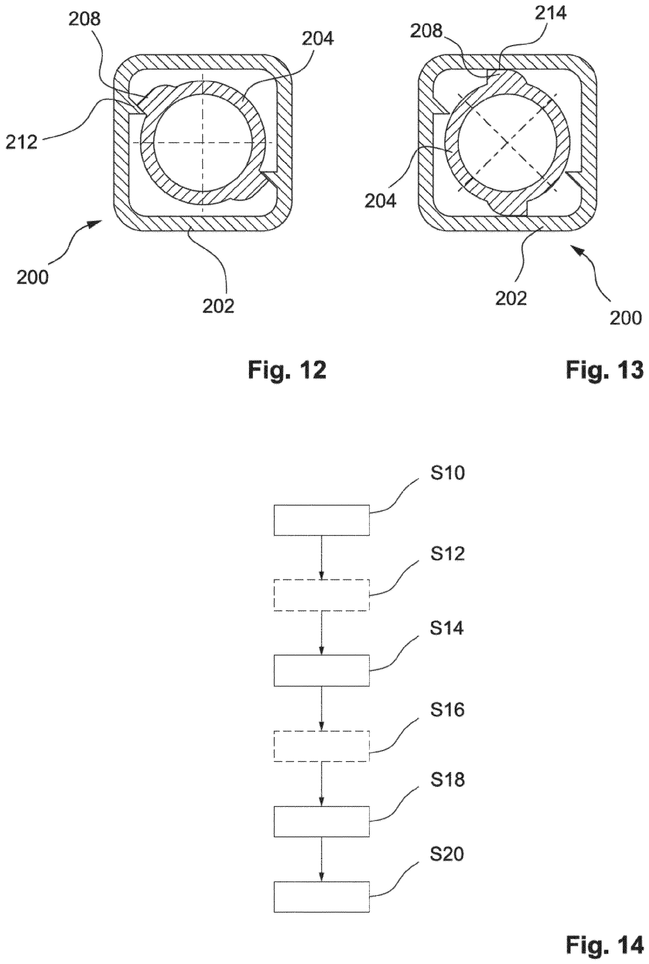

In diesem Zusammenhang veranschaulichen die Figuren 12 und 13 eine weitere beispielhafte Ausgestaltung eines Klemmelement 200. Beispielhaft kann das Klemmelement 200 als Alternative zu einem der Klemmelemente 140, 142 vorgesehen sein. Fig. 12 zeigt einen Freigabezustand. Fig. 13 zeigt einen Klemmzustand des Klemmelements 200.

Das Klemmelement 200 ist zwischen einem Schubelement 202 (vergleiche die Schubelemente 52, 54, 56) und einem Übertragungselement 204 (vergleiche die Übertragungselemente 84, 86, 88) ausgebildet. Das Klemmelement 200 umfasst eine Exzenterkontur 208, die beispielhaft zwei um 180° versetzte Erhebungen am Übertragungselement 204 umfasst. Die Exzenterkontur 208 kann auch als Exzenternocken bezeichnet werden. In der Freigabestellung gemäß Fig. 12 liegt die Exzenterkontur 208 an einem Anschlag 212 an, der dem Schubelement 202 zugeordnet ist.

Durch eine Rotationsbewegung des Übertragungselement 204 mit den Exzenterkonturen 208 (im Ausführungsbeispiel im Uhrzeigersinn) wird das Klemmelement 200 in den Klemmzustand überführt. In Fig. 13 bilden die Exzenterkonturen 208 durch eine bündige oder weitgehend bündige Anlage am Schubelement 202 einen Anschlag 214 für den Klemmzustand aus. Es versteht sich, dass der Anschlag 214 auch anderweitig gestaltet sein kann und insbesondere erhabene Gestaltelemente beim Schubelement 202 umfassen kann.

Die erhabenen Exzenterkonturen 208 können als hinreichend steife Konturen gestaltet sein. Auf diese Weise ergibt sich die Haltekraft im Klemmzustand in erster Linie durch die erzeugte Haftreibung. Die erhabenen Exzenterkonturen 208 können jedoch auch zumindest teilweise elastisch (nachgiebig) gestaltet sein. Auf diese Weise ergibt sich die Haltekraft im Klemmzustand zumindest teilweise durch eine Federkraft der Exzenterkonturen 208. Gewünschte Eigenschaften der Exzenterkonturen 208 können beispielsweise über die Wahl des Materials beeinflusst werden.

Die Figuren 12 und 13 veranschaulichen in Ergänzung zu den Figuren 5-11, dass der Übergang zwischen dem Freigabezustand und dem Klemmzustand zum einen durch Exzenterkonturen 208 ermöglicht wird und zum anderen aufgrund der bewusst unterschiedlichen Querschnittsgestaltung der Schubelemente 202 und der Übertragungselemente 204 ermöglicht wird. In einer solchen Konstellation genügt eine Relativrotation zwischen den Schubelementen 202 und den Übertragungselement 204 mit einem überschaubaren Schwenkwinkel (etwa 45° oder gar weniger), um die gewünschte Klemmung oder Freigabe herbeizuführen. Dies ist nicht einschränkend zu verstehen. Es versteht sich, dass insbesondere die Schubelemente 202 andere Querschnittsprofile als Vierkantprofile (quadratischer Querschnitt oder rechteckiger Querschnitt) aufweisen können.

Mit Bezugnahme auf Fig. 14 wird anhand eines schematisch stark vereinfachten Flussdiagramms eine beispielhafte Ausgestaltung eines Verfahrens zur Positionierung eines medizinischen Instruments veranschaulicht. Das Verfahren eignet sich für medizinische Instrumente, die zur Beobachtung des Körpers eines Patienten von außerhalb des Körpers ausgebildet sind, bei denen also keine Zugangsöffnung zum Körper erforderlich ist.

Das Verfahren umfasst einen Schritt S10, der die Bereitstellung einer Haltevorrichtung gemäß zumindest einer der hierin beschriebenen Ausgestaltungen umfasst. Es kann sich ein optionaler Schritt S12 anschließen, der die Befestigung der Haltevorrichtung an einem Gestell im Umfeld einer Patientenauflage beinhaltet. Es kann sich um eine temporäre oder um eine dauerhafte Befestigung handeln.

Ein weiterer Schritt S14 betrifft die Befestigung eines medizinischen Instruments an einem Instrumentenhalter der Haltevorrichtung. Bei dem Instrument handelt sich beispielhaft um ein Beobachtungsinstrument. Es kann sich ein optionaler Schritt S16 anschließen, der beispielsweise dann erforderlich ist, wenn sich die Klemmelemente der Haltevorrichtung im Klemmzustand befinden. Im Schritt S16 werden einige oder sämtliche der Klemmelemente in den Freigabezustand überführt, um Bewegungsfreiheitsgrade der Haltevorrichtung freizugeben.

In einem Schritt S18 erfolgt die Positionierung des Instruments. Dies betrifft einerseits die Platzierung des Instruments an einem bestimmten Ort (beispielsweise an einer bestimmten Koordinate in einer Ebene) und zum anderen eine gewünschte Ausrichtung des Instruments. Die erstgenannte Platzierung nutzt beispielhaft den Rotationsfreiheitsgrad des Drehgelenks sowie den translatorischen Freiheitsgrad des Schubgelenks der Haltevorrichtung. Die Ausrichtung kann eine Schwenkorientierung des Instruments unter Nutzung des Kugelgelenks der Haltevorrichtung betreffen. Ferner kann die Ausrichtung eine Rotation des Instruments um dessen Längsachse und gegebenenfalls eine Bewegung entlang der Längsachse betreffen.

Wenn das Gerät in der gewünschten Position und Ausrichtung platziert ist, kann die Haltevorrichtung in einem nachfolgenden Schritt S20 blockiert werden, indem Klemmelemente der Haltevorrichtung in einem Klemmzustand überführt werden. Auf diese Weise ist das Instrument hinreichend fixiert und lagegesichert. Es kann sich eine medizinische Prozedur unter Nutzung des Instruments anschließen.

Im Falle einer Neupositionierung oder eines Wechsels des Instruments können zumindest einige der Schritte des Verfahrens erneut durchlaufen werden. Vorteilhaft ist die simultane Aktivierung oder Deaktivierung der Klemmelemente, zumindest in beispielhaften Ausgestaltungen, wodurch die gesamte Haltevorrichtung mit nur einer Aktion bedarfsweise in den Freigabezustand oder den Klemmzustand überführt werden kann. Die eigentliche Positionierung und Ausrichtung des Instruments kann händisch erfolgen. Die Ansteuerung der Klemmelemente kann über einen Antrieb erfolgen, beispielsweise über einen motorischen Antrieb.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Offenbarung zu verlassen.

Die Offenbarung betrifft eine Haltevorrichtung 12 für medizinische Instrumente 14, mit einer proximalen Basis 20 zur Aufnahme an einem Gestell 16, die ein erstes Gelenk 30 umfasst, einem distalen Instrumentenhalter 22, der ein zweites Gelenk 32 umfasst, zumindest zwei Schubelementen 52, 54, 58 zwischen der Basis 20 und dem Instrumentenhalter 22, und zumindest zwei Klemmelementen 138, 140, 142, 144, die durch zumindest ein Übertragungselement 84, 86, 88 betätigbar sind, die gemeinsam in einem Klemmzustand und einem Freigabezustand betreibbar sind, und die dazu ausgebildet sind, Gelenke der Haltevorrichtung 12 im Klemmzustand zu blockieren, wobei die zumindest zwei Schubelemente 52, 54, 58 ein Schubgelenk 24 bilden und relativ zueinander entlang einer Längsachse 132 translatorisch verfahrbar sind, und wobei die zumindest zwei Schubelemente 52, 54, 58 relativ zueinander teleskopierbar und drehfest miteinander gekoppelt sind. Die Offenbarung betrifft ferner ein medizinisches System, eine Verwendung einer Haltevorrichtung, sowie ein Verfahren zur Positionierung eines medizinischen Instruments.

## Patentansprüche

1. Haltevorrichtung (12) für medizinische Instrumente (14), insbesondere endoskopische Instrumente (14), die Folgendes aufweist:
- eine proximale Basis (20) zur Aufnahme an einem Gestell (16), die ein erstes Gelenk (30) umfasst,
- einen distalen Instrumentenhalter (22), der ein zweites Gelenk (32) umfasst,
- zumindest zwei Schubelemente (52, 54, 58) zwischen der Basis (20) und dem Instrumentenhalter (22), und
- zumindest zwei Klemmelemente (138, 140, 142, 144), die durch zumindest ein Übertragungselement (84, 86, 88) betätigbar sind, die gemeinsam in einem Klemmzustand und einem Freigabezustand betreibbar sind, und die dazu ausgebildet sind, Gelenke der Haltevorrichtung (12) im Klemmzustand zu blockieren,
wobei die zumindest zwei Schubelemente (52, 54, 58) ein Schubgelenk (24) bilden und relativ zueinander entlang einer Längsachse (132) translatorisch verfahrbar sind, und wobei die zumindest zwei Schubelemente (52, 54, 58) relativ zueinander teleskopierbar und drehfest miteinander gekoppelt sind.

2. Haltevorrichtung (12) nach Anspruch 1, wobei ein gemeinsamer Antrieb (94) für die zumindest zwei Klemmelemente (138, 140, 142, 144) vorgesehen ist, insbesondere ein einziger Antrieb (94), der die zumindest zwei Klemmelemente (138, 140, 142, 144) simultan betätigt.

3. Haltevorrichtung (12) nach Anspruch 2, wobei der Antrieb (94) eine Antriebswelle (102) umfasst, die mit zumindest einem der Klemmelemente (140, 142, 144) zusammenwirkt, wobei der Antrieb (94) ferner ein Lagerstück (100) aufweist, das beim Antreiben der Antriebswelle (102) ein resultierendes Gegenmoment aufnimmt, und wobei das Lagerstück (100) mit zumindest einem anderen der Klemmelemente (138) zusammenwirkt.

4. Haltevorrichtung (12) nach Anspruch 3, wobei die Antriebswelle (102) parallel zur Längsachse (132) orientiert ist, und wobei das Lagerstück (100) mit einem proximalen Klemmelement (138) gekoppelt ist, das dem ersten Gelenk (30) zugeordnet ist.

5. Haltevorrichtung (12) nach einem der Ansprüche 2-4, wobei der Antrieb (94) mit zumindest zwei teleskopierbaren Übertragungselementen (84, 86, 88) gekoppelt ist, wobei die Anzahl der Übertragungselemente (84, 86, 88) an die Anzahl der Schubelemente (52, 54, 58) angepasst ist, und wobei die Übertragungselemente (84, 86, 88) eine durch den Antrieb (94) induzierte Drehbewegung übertragen, um die zumindest zwei Klemmelemente (138, 140, 142, 144) in den Klemmzustand oder den Freigabezustand zu überführen.

6. Haltevorrichtung (12) nach einem der Ansprüche 2-5, wobei der Antrieb (94) derart mit zwei benachbarten Übertragungselementen (84, 86) gekoppelt ist, dass die benachbarten Übertragungselemente (84, 86) bei einer Bewegung des Antriebs (94) gegensinnig rotiert werden.

7. Haltevorrichtung (12) nach einem der Ansprüche 2-6, wobei die Schubelemente (52, 54, 58) und die Übertragungselemente (84, 86, 88) jeweils als Hohlprofilkörper (66, 90) gestaltet sind, wobei die Übertragungselemente (84, 86, 88) innerhalb der Schubelemente (52, 54, 58) angeordnet sind, und wobei der Antrieb (94) ein patronenartiges Gehäuse (98) aufweist, das drehfest mit einem der Übertragungselemente (84, 86, 88) gekoppelt ist, insbesondere mit einem proximalen Übertragungselement (84).

8. Haltevorrichtung (12) nach einem der Ansprüche 2-7, wobei drei oder mehr Übertragungselemente (84, 86, 88) vorgesehen sind, von denen zumindest zwei benachbarte Übertragungselemente (86, 88), insbesondere zwei distale Übertragungselemente (86, 88), drehfest miteinander gekoppelt und relativ zueinander translatorisch verfahrbar sind.

9. Haltevorrichtung (12) nach einem der Ansprüche 1-8, umfassend zumindest drei Schubelemente (52, 54, 58), die ein Schubgelenk (24) bilden und teleskopierbar sind, wobei Querschnittsprofile der Schubelemente (52, 54, 58) ausgehend von der proximalen Basis (20) hin zum distalen Instrumentenhalter (22) abgestuft sind und kleiner werden.

10. Haltevorrichtung (12) nach einem der Ansprüche 1-9, wobei die zumindest zwei Schubelemente (52, 54, 58) ein Kastenprofil aufweisen, insbesondere ein Vierkantprofil, und wobei die Kastenprofile der Schubelemente (52, 54, 58) derart aneinander angepasst sind, dass die Schubelemente (52, 54, 58) entlang der Längsachse (132) teleskopierbar und verdrehsicher miteinander gekoppelt sind.

11. Haltevorrichtung (12) nach einem der Ansprüche 1-10, wobei zumindest die den Schubelementen (52, 54, 58) zugeordneten Klemmelemente (140, 142) rotatorisch betätigbar sind und die Schubelemente (52, 54, 58) mit dem zumindest einen Übertragungselement (84, 86, 88) in einer gegebenen translatorischen Position klemmen.

12. Haltevorrichtung (12) nach Anspruch 11, wobei zumindest eines der Klemmelemente (138, 140, 142, 144) einen Exzenter (118, 124, 150, 160, 208) aufweist, wobei zumindest ein Exzenter als Federexzenter (150, 160) gestaltet ist und/oder Exzenterkonturen (118, 124, 208) aufweist, und wobei der Exzenter (118, 124, 150, 160, 208) bei einer Relativrotation zwischen dem zumindest einen Übertragungselement (84, 86, 88) und den Schubelementen (52, 54, 58) eine kraftschlüssige Fixierung bewirkt.

13. Medizinisches System, insbesondere endoskopisches System, umfassend eine Haltevorrichtung (12) nach einem der Ansprüche 1-12 sowie ein medizinisches Instrument (14), das am distalen Instrumentenhalter (22) aufgenommen ist, wobei das medizinische Instrument (14) den distalen Instrumentenhalter (22) durchragt, und wobei das medizinische Instrument (14) entlang einer Instrumentenachse (46) beweglich ist, wenn ein dem zweiten Gelenk (32) zugeordnetes Klemmelement (144) im Freigabezustand ist.

14. Verwendung einer Haltevorrichtung (12) nach einem der Ansprüche 1-12 zur Positionierung eines medizinischen Instruments (14), insbesondere eines endoskopischen Instruments.

15. Verfahren zur Positionierung eines medizinischen Instruments, insbesondere eines endoskopischen Instruments (14), mit den Schritten:
- Bereitstellung einer Haltevorrichtung (12) nach einem der Ansprüche 1-12,
- Befestigung eines medizinischen Instruments (14) am distalen Instrumentenhalter (22),
- Positionieren der Haltevorrichtung (12) mit dem Instrument (14) im Freigabezustand der zumindest zwei Klemmelemente (138, 140, 142, 144), und
- Blockieren der Haltevorrichtung (12) im Klemmzustand der zumindest zwei Klemmelemente (138, 140, 142, 144).
